# EUROPEAN PATENT APPLICATION

(11) **EP 2 455 754 A2**
(43) Date of publication of application: **23.05.2012**
(21) Application number: 11189305.3
(22) Date of filing: 16.11.2011
(51) Int. Cl.: G01N 29/024, G01N 29/07, G01N 29/06, A61B 8/00

(54) **Ultrasound System and Method for Providing Enlarged Image**

(30) Priority: 18.11.2010 KR 20100114842
(71) Applicant: Samsung Medison Co., Ltd., Nam-myun Hongchun-gun Kangwon do 250-875 (KR)
(72) Inventor: Kim, Sung Hee, 135-851 Seoul (KR)
(74) Representative: Schmid, Wolfgang

(57) **Abstract**

Embodiments of providing an enlarged image, without degradation of image quality are disclosed. The ultrasound system includes an ultrasound data acquisition unit, a user input unit and a processor. The ultrasound data acquisition unit is configured to acquire ultrasound data representative of a target object. The user input unit is configured to receive user input information. The processor is configured to extract ultrasound data corresponding to the user input information from the acquired ultrasound data, perform a decimation process on the extracted ultrasound data based on a zoom ratio corresponding to the user input information and form an enlarged image corresponding to the zoom ratio by using the decimated ultrasound data.

## Description

### CROSS-REFERENCE TO RELATED APPLICATIONS

The present application claims priority from Korean Patent Application No. 10-2010-0114842 filed on November 18, 2010.

### TECHNICAL FIELD

The present invention relates to ultrasound systems, and more particularly to an ultrasound system and method for providing an enlarged image.

### BACKGROUND

Since an ultrasound system has noninvasive and nondestructive characteristics, it has been widely used in the medical fields to acquire information on the inner parts of a target object. The ultrasound system may provide doctors with high-resolution images of the inner parts of a target object without any surgical operation. Hence, the ultrasound system is very important in the medical fields.

The ultrasound system forms an ultrasound image by transmitting an ultrasound signal to a target object and receiving an ultrasound echo signal reflected from the target object through an ultrasound probe.

Further, the ultrasound system provides an image zoom function to enlarge an ultrasound image. Specifically, when a region of interest, which is to be enlarged, is set in an ultrasound image, the ultrasound system provides read zoom and write zoom functions that enlarge an image corresponding to the region of interest. In particular, the read zoom function is to enlarge an ultrasound image during an output procedure for display on a display unit after the scan conversion.

Conventionally, when the read zoom function is selected, an image is enlarged without adjusting image data after the scan conversion. Thus, the image quality of the enlarged image may be degraded.

### SUMMARY

The present invention is directed to an ultrasound system and method for providing an enlarged image without any degradation of image quality by performing data processing corresponding to a read zoom function upon ultrasound data within a region of interest before scan conversion.

In accordance with an embodiment of the present invention, an ultrasound system includes: an ultrasound data acquisition unit configured to transmit an ultrasound signal to a target object and receive an ultrasound echo signal reflected from the target object to acquire ultrasound data; a user input unit configured to receive user input information; and a processor connected to the ultrasound data acquisition unit and the user input unit, the processor being configured to extract ultrasound data corresponding to the user input information from the acquired ultrasound data, perform a decimation process on the extracted ultrasound data based on a zoom ratio corresponding to the user input information, and form an enlarged image corresponding to the zoom ratio by using the decimated ultrasound data.

In accordance with another embodiment of the present invention, a method of providing an enlarged image includes: a) transmitting an ultrasound signal to a target object and receiving an ultrasound echo signal reflected from the target object to acquire ultrasound data; b) extracting ultrasound data corresponding to first user input information from the acquired ultrasound data; c) performing a decimation process on the extracted ultrasound data based on a zoom ratio corresponding to second user input information; and d) forming an enlarged image corresponding to the zoom ratio by using the decimated ultrasound data.

### BRIEF DESCRIPTION OF THE DRAWINGS

FIG. 1 is a block diagram of an ultrasound system in accordance with an embodiment of the present invention.
FIG. 2 is a block diagram of an ultrasound data acquisition unit in accordance with an embodiment of the present invention.
FIG. 3 is an exemplary diagram showing an ultrasound image and a region of interest in accordance with an embodiment of the present invention.
FIG. 4 is a flowchart showing a procedure of forming an enlarged image in accordance with a first embodiment of the present invention.
FIG. 5 is an exemplary diagram showing ultrasound data and a decimation factor in accordance with an embodiment of the present invention.
FIG. 6 is a flowchart showing a procedure of forming an enlarged image in accordance with a second embodiment of the present invention.
FIG. 7 is an exemplary diagram showing ultrasound data and an ultrasound image in accordance with an embodiment of the present invention.
FIGS. 8 and 9 are exemplary diagrams showing displaying of an ultrasound image and an enlarged image in a dual mode in accordance with an embodiment of the present invention.

### DETAILED DESCRIPTION

Hereinafter, exemplary embodiments of the present invention will be described with reference to the accompanying drawings.

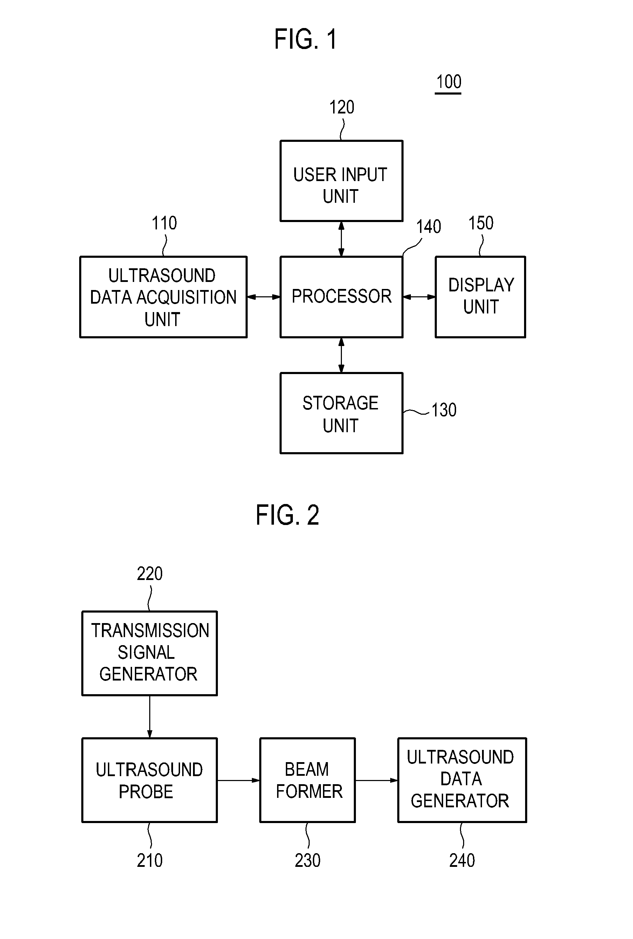

FIG. 1 is a block diagram of an ultrasound system in accordance with an embodiment of the present invention. Referring to FIG. 1, the ultrasound system 100 includes an ultrasound data acquisition unit 110, a user input unit 120, a storage unit 130, a processor 140 and a display unit 150.

The ultrasound data acquisition unit 110 acquires ultrasound data by transmitting an ultrasound signal to a target object and receiving an ultrasound echo signal reflected from the target obj ect.

FIG. 2 is a block diagram of the ultrasound data acquisition unit in accordance with an embodiment of the present invention. Referring to FIG. 2, the ultrasound data acquisition unit 110 includes an ultrasound probe 210, a transmission signal generator 220, a beam former 230 and an ultrasound data generator 240.

The ultrasound probe 210 includes a plurality of transducer elements (not shown) for reciprocal conversion between an electric signal and an ultrasound signal. The ultrasound probe 210 transmits an ultrasound signal to a target object along a plurality of scan lines and receives an ultrasound echo signal reflected from the target object to thereby form a reception signal. The reception signal is an analog signal. The ultrasound probe 210 includes a convex probe, a linear probe or the like. However, the ultrasound probe 210 is not limited thereto.

The transmission signal generator 220 controls the transmission of the ultrasound signal according to a desired ultrasound image. The transmission signal generator 220 generates a transmission signal to be applied to the ultrasound probe 210, considering the transducer elements and focusing points. The ultrasound image includes a brightness mode (B mode) image. However, the ultrasound image is not limited thereto.

The beam former 230 performs an analog-to-digital conversion on the reception signal provided from the ultrasound probe 210 to form a digital signal. In addition, the beam former 230 further performs receive-focusing upon the digital signal by considering the transducer elements and the focusing points to form a receive-focused signal.

The ultrasound data generator 240 generates ultrasound data corresponding to the ultrasound image by using the receive-focused signal provided from the beam former 230. The ultrasound data includes radio frequency (RF) data. However, the ultrasound data is not limited thereto. Further, the ultrasound data generator 240 may perform a variety of signal processing (e.g., gain control) on the receive-focused signal to generate the ultrasound data.

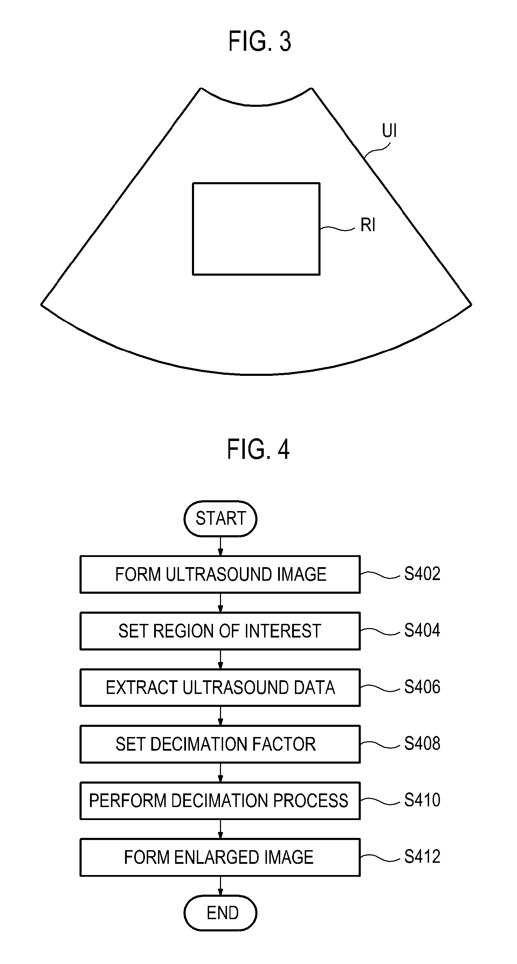

Referring back to FIG. 1, the user input unit 120 receives user input information. In this embodiment, as shown in FIG. 3, the user input information includes first user input information and second user input information. The first user input information is used to set a region of interest RI, which is to be enlarged, in an ultrasound image UI. The second input information is used to set a zoom ratio of an enlarged image. The user input unit 120 includes a control panel, a track ball, a mouse, a keyboard or the like. However, the user input unit 120 is not limited thereto.

The storage unit 130 stores the ultrasound data acquired by the ultrasound data acquisition unit 110. Further, the storage unit 130 may store the user input information received from the user input unit 120.

The processor 140 is connected to the ultrasound data acquisition unit 110, the user input unit 120 and the storage unit 130. The processor 140 includes a central processing unit (CPU), a microprocessor, a graphic processing unit (GPU) or the like. However, the processor 140 is not limited thereto.

FIG. 4 is a flowchart showing a procedure of providing an enlarged image in accordance with a first embodiment of the present invention. Referring to FIG. 4, the processor 140 forms the ultrasound image UI, as shown in FIG. 3, using the ultrasound data provided from the ultrasound data acquisition unit 110 at S402. The ultrasound image UI may be displayed on the display unit 150. Thus, the user input unit 120 may allow the user to set the region of interest RI in the ultrasound image UI displayed on the display unit 150.

Although it has been described in the above embodiment that the processor 140 forms the ultrasound image using the ultrasound data provided from the ultrasound data acquisition unit 110, the invention is not limited thereto. The processor 140 may form the ultrasound image using the ultrasound data stored in the storage unit 130.

When the processor 140 receives the user input information (that is, the first user input information) from the user input unit 120, the processor 140 sets the region of interest RI in the ultrasound image UI as shown in FIG. 3 based on the first user input information at S404 and searches the storage unit 130 to extract ultrasound data corresponding to the region of interest RI at S406.

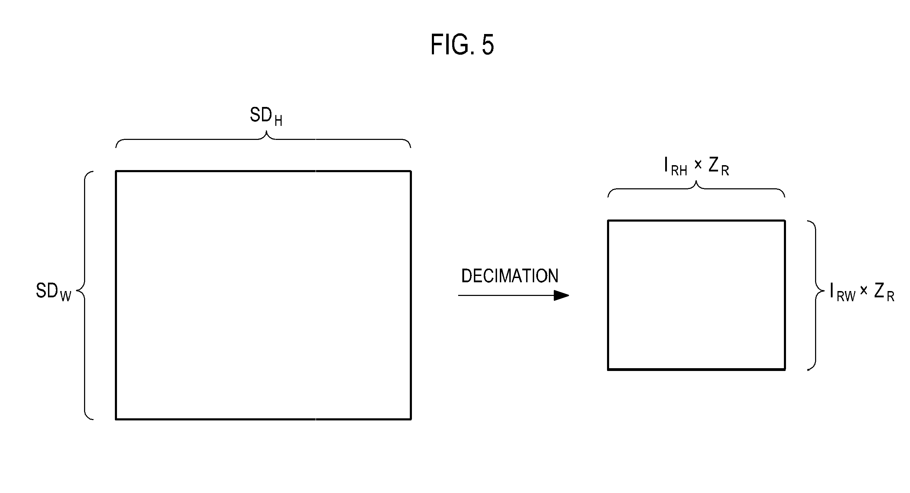

When the processor 140 receives the user input information (that is, the second user input information) from the user input unit 120, the processor 140 sets a decimation factor for performing a decimation process of sampling the ultrasound data extracted from the storage unit 130 based on the second user input information at S408. For example, as shown in FIG. 5, the processor 140 multiplies a preset decimation factor (that is, a screen size (I_{RH}, I_{RW}) of the display unit 150) by a zoom ratio (Z_{R}) corresponding to the second user input information to reset a decimation factor (I_{RH}×Z_{R,} I_{RW}×Z_{R})_{.}

The processor 140 performs a decimation process on the ultrasound data extracted from the storage unit 130 based on the reset decimation factor at S410. For example, as shown in FIG. 5, the processor 140 performs a decimation process on the extracted ultrasound data (SD_{H}×SD_{W}) based on the reset decimation factor (I_{RH}×Z_{R}, I_{RW}×Z_{R}).

The processor 140 performs scan conversion on the decimated ultrasound data to form an enlarged image at S412.

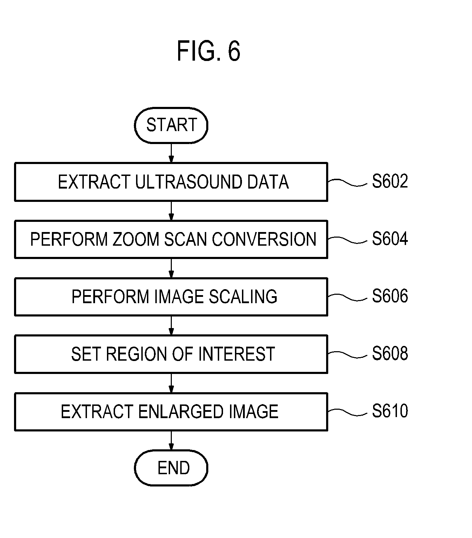

FIG. 6 is a flowchart showing a procedure of providing an enlarged image in accordance with a second embodiment of the present invention. Referring to FIG. 6, the processor 140 extracts ultrasound data stored in the storage unit 120 at S602.

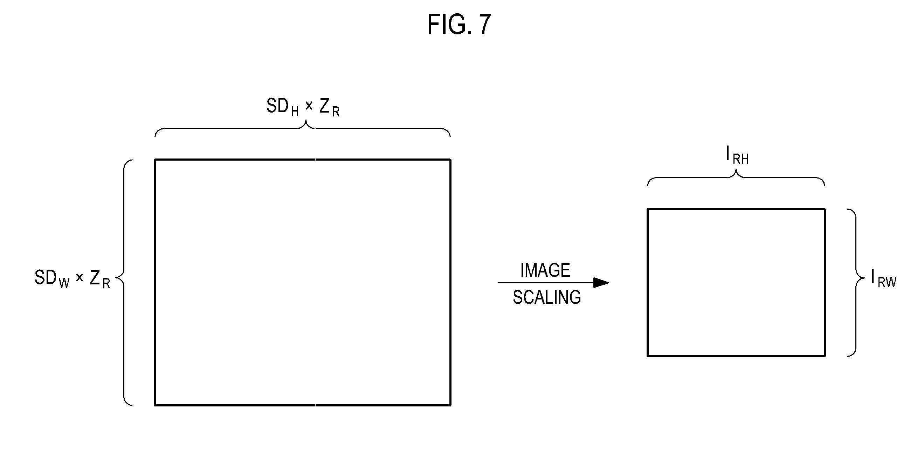

When the processor 140 receives the user input information (that is, the second user input information) from the user input unit 120, the processor 140 performs scan conversion upon the extracted ultrasound data for enlargement by a zoom ratio corresponding to the second user input information to form an ultrasound image at S604. For example, as shown in FIG. 7, the processor 140 performs scan conversion upon the extracted ultrasound data (SD_{H}×SD_{W}) for enlargement by a zoom ratio (Z_{R}) corresponding to the second user input information to form an ultrasound image DUI.

Although it has been described in the above embodiment that the zoom scan conversion is performed on the ultrasound data stored in the storage unit 120, the invention is not limited thereto. The zoom scan conversion may be performed on the ultrasound data provided from the ultrasound data acquisition unit 110.

The processor 140 performs image scaling on the ultrasound image to reduce a size thereof at S606. The reduced ultrasound image may be displayed on the display unit 150. Thus, the user input unit 120 may allow the user to set the region of interest RI in the ultrasound image UI displayed on the display unit 150. For example, as shown in FIG. 7, the processor 140 performs image scaling on the zoom-scan-converted ultrasound image data by a zoom ratio corresponding to the screen size (I_{RH}, I_{RW}) of the display unit 150 to reduce the size of the ultrasound image.

When the processor 140 receives the user input information (that is, the first user input information) from the user input unit 120, the processor 140 sets the region of interest in the zoom-scan-converted ultrasound image based on the first user input information at S608. For example, the processor 140 sets the region of interest in the reduced ultrasound image based on the first user input information, enlarges the set region of interest by a zoom ratio corresponding to the screen size (I_{RH}, I_{RW}) of the display unit 150, and sets the enlarged region of interest in the zoom-scan-converted ultrasound image.

The processor 140 extracts an enlarged image corresponding to the enlarged region of interest from the zoom-scan-converted ultrasound image at S610.

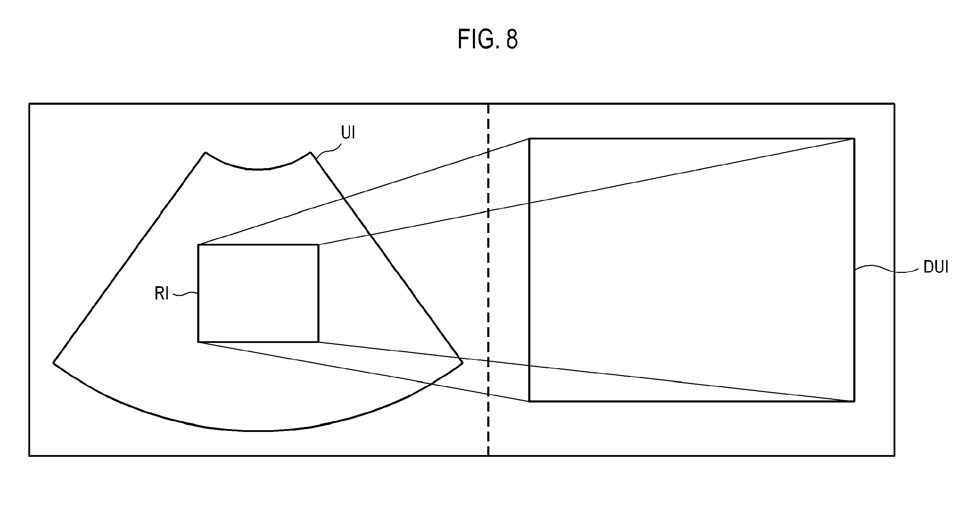

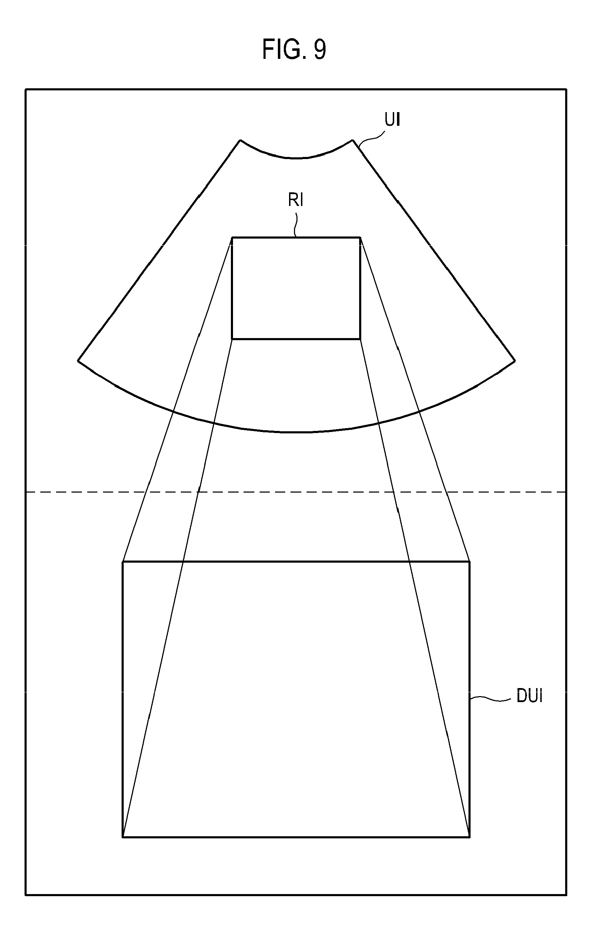

Referring again to FIG. 1, the display unit 150 displays the ultrasound image formed by the processor 140. Further, the display unit 150 displays the enlarged image formed by the processor 140. For example, as shown in FIG. 8 or 9, the display unit 150 includes a wide-view display. Thus, the ultrasound image UI and the enlarged image DUI may be displayed in the same size, as shown in FIG. 8 or 9.

While the invention has been shown and described with reference to exemplary embodiments thereof, it will be understood by those skilled in the art that various changes in form and details may be made therein without departing from the scope of the invention as defined by the appended claims.

## Claims

1. An ultrasound system, comprising:
an ultrasound data acquisition unit configured to transmit an ultrasound signal to a target object and receive an ultrasound echo signal reflected from the target obj ect to acquire ultrasound data;
a user input unit configured to receive user input information; and
a processor connected to the ultrasound data acquisition unit and the user input unit, the processor being configured to extract ultrasound data corresponding to the user input information from the acquired ultrasound data, perform a decimation process on the extracted ultrasound data based on a zoom ratio corresponding to the user input information, and form an enlarged image corresponding to the zoom ratio by using the decimated ultrasound data.

2. The ultrasound system of Claim 1, wherein the user input information comprises:
first user input information configured to set a region of interest to be enlarged in an ultrasound image corresponding to the ultrasound data; and
second user input information configured to set the zoom ratio.

3. The ultrasound system of Claim 1, wherein the processor is configured to perform the decimation process on the extracted ultrasound data to set a decimation factor, and wherein the processor is configured to perform the decimation process on the extracted ultrasound data based on the set decimation factor.

4. The ultrasound system of Claim 3, wherein the processor is configured to multiply the zoom ratio by a preset decimation factor to set the decimation factor.

5. The ultrasound system of Claim 1, wherein the processor is further configured to perform zoom scan conversion on the ultrasound data in the zoom ratio corresponding to the user input information to form an enlarged ultrasound image and to extract an enlarged image corresponding to the user input information from the enlarged ultrasound image.

6. The ultrasound system of Claim 5, wherein the user input information comprises:
first user input information configured to set the region of interest; and
second user input information configured to set the zoom ratio.

7. The ultrasound system of Claim 5, wherein:
the processor is configured to perform image scaling on the enlarged ultrasound image to reduce a size of the enlarged ultrasound image;
the processor is configured to set the region of interest in the reduced ultrasound image, based on the user input information;
the processor is configured to enlarge the region of interest by the zoom ratio and set the enlarged region of interest in the enlarged ultrasound image; and the processor is configured to extract the enlarged image corresponding to the enlarged region of interest from the enlarged ultrasound image.

8. A method of providing an enlarged image, comprising:
a) transmitting an ultrasound signal to a target object and receiving an ultrasound echo signal reflected from the target object to acquire ultrasound data;
b) extracting ultrasound data corresponding to first user input information from the acquired ultrasound data;
c) performing a decimation process on the extracted ultrasound data based on a zoom ratio corresponding to second user input information; and
d) forming an enlarged image corresponding to the zoom ratio by using the decimated ultrasound data.

9. The method of Claim 8, wherein the step b) comprises:
forming an ultrasound image using the ultrasound data;
receiving the first user input information for seting a region of interest in the ultrasound image; and
extracting ultrasound data corresponding to the region of interest from the ultrasound data.

10. The method of Claim 8, wherein the step c) comprises:
c1) performing the decimation process on the extracted ultrasound data to set a decimation factor; and
c2) performing the decimation process on the extracted ultrasound data based on the set decimation factor.

11. The method of Claim 10, wherein the step c1) comprises multiplying the zoom ratio by a preset decimation factor to set the decimation factor.

12. The method of Claim 8, further comprising:
performing a zoom scan conversion on the acquired ultrasound data by the zoom ratio corresponding to first user input information to form an enlarged ultrasound image ; and
extracting an enlarged image corresponding to second user input information from the enlarged ultrasound image.

13. The method of Claim 12, wherein the extracting step comprises:
performing image scaling on the enlarged ultrasound image to reduce a size of the enlarged ultrasound image;
setting a region of interest in the enlarged ultrasound image based on the second user input information;
setting the enlarged region of interest in the enlarged ultrasound image by enlarging the region of interest by the zoom ratio; and
extracting the enlarged image corresponding to the enlarged region of interest from the enlarged ultrasound image.
